## Europäisches Patentamt

(19) **European Patent Office**

**Office européen des brevets**

(11) Publication number: **0 045 623**
**B1**

(12) **EUROPEAN PATENT SPECIFICATION**

(45) Date of publication of patent specification: **30.01.85**

(21) Application number: **81303474.1**

(22) Date of filing: **28.07.81**

(51) Int. Cl.⁴: **G 01 N 33/18**

(54) **Apparatus and method for measuring the toxicity of pollutants to aquatic living organisms.**

(30) Priority: **04.08.80 GB 8025348**

(43) Date of publication of application:
**10.02.82 Bulletin 82/06**

(45) Publication of the grant of the patent:
**30.01.85 Bulletin 85/05**

(84) Designated Contracting States:
**AT BE CH DE FR IT LI LU NL SE**

(56) References cited:
**DE-A-2 415 771**
**DE-A-2 431 918**
**FR-A-2 331 024**
**FR-A-2 356 141**
**US-A-3 731 807**

(73) Proprietor: **EUROPEAN ATOMIC ENERGY COMMUNITY (EURATOM)**
**Batiment Jean Monnet Plateau du Kirchberg**
**Boîte Postale 1907**
**Luxembourg (LU)**

(72) Inventor: **Ghetti, Pier Francesco, Prof.**
**via Maggio No. 11**
**Parma (IT)**
Inventor: **Bellavere, Carlo, Dr**
**.via Masini**
**Parma (IT)**
Inventor: **Ravera, Oscar**
**via Rancio 13**
**Luvinate Varese (IT)**

(74) Representative: **Baverstock, Michael George Douglas et al**
**BOULT, WADE & TENNANT 27 Furnival Street**
**London, EC4A 1PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to an apparatus and method for measuring the toxicity of pollutants to aquatic living organisms in which the effect of a presumed toxic substance on aquatic living organisms is measured at different concentrations of the toxic substance.

The Applicants are aware of United Kingdom Patent Specification No 1318911 and DE—A—2415771. DE—A—2415771 relates to the determination of the toxicity of waste water for metabolic processes as expressed by oxygen consumption.

The measuring arrangement required to determine the biochemical oxygen requirement is an indicating or recording differential pressure measuring instrument which is connected simultaneously to two closed systems that are filled with the substance of a water sample of varying dilution and whose recorded water volumes are in inverse proportion to the dilution. If no deflection is noted on the measuring instrument during the period of consumption, the biochemical oxygen requirement is the same in both systems and there is no toxic effect. There is toxicity, however, if the less diluted sample has a lower oxygen requirement.

The ability of the prior art apparatus to measure the effects on one or more organisms simultaneously in a continuous liquid flow has not been disclosed.

In accordance with the invention an apparatus has been devised which permits the performance of such toxicity tests not only in the laboratory, but also in the field, to test, for example, river waters, lake waters and industrial wastes.

Accordingly the present invention is characterised in that the apparatus is adapted to measure the effects on one or more organisms simultaneously in a continuous liquid flow, the apparatus comprising a tank for storing the toxic substance in liquid form and a tank for storing unpolluted dilution water, two headers connecting the respective tanks to a peristaltic pump, the pump being adapted by having flexible tubes of different diameters simultaneously to supply preselected differently proportioned amounts of toxic liquid and dilution water, and means for mixing and distributing said differently proportioned amounts of toxic liquid and dilution water through siphons to tanks containing the organisms to be tested.

The invention also provides a method of measuring the toxicity of pollutants to aquatic living organisms which method comprises operating the above apparatus and observing the effects on the organisms in the tanks containing them.

Although the following description refers to three different species of organisms studies in four different concentrations of toxic liquid and in clean water, the flexibility of the apparatus of the invention permits a study also of, for example, one kind of organism with seven different concentrations of toxic liquid with two replicates or, for example, two kinds of organism with six different concentrations of toxic substance at the same time.

The organisms used as examples in this description are those commonly used in toxicology tests, that is: Daphnia magna (Crustacea), Biomphalaria glabrata (Mollusca) and the Brachydanio rerio (Pisces).

The operation characteristics of the apparatus have been chosen in such a way as to make it possible to carry out the toxicity tests in conditions of continuous flow, with more than one species at a time, and with different concentrations of the toxic liquid.

The concentrations of the toxic liquid used in experiments described were 100%, 75%, 50% and 25% of the initial concentration of the toxic liquid used, and clean water, otherwise called the control reference.

An embodiment of an apparatus according to the invention will now be described by way of example with reference to the accompanying drawings in which:—

Figure 1 is a general diagram of the apparatus;

Figure 2 is vertical section of a header used in the apparatus;

Figure 3(a) is a horizontal section through the means for mixing and distributing proportional amounts of toxic liquid and dilution water in the apparatus, referred to hereafter in this description as a diffuser;

Figures 3(b) and 3(c) are vertical sections at A—A' and B—B' of Figure 3(a).

Figure 4(a) is a vertical section through a siphon of the apparatus;

Figure 4(b) is a plan view of the siphon of Figure 4(a);

Figure 5(a) is a horizontal section of a tank suitable for containing an aquatic organism of the Daphnia type;

Figure 5(b) is a vertical section of the tank of Figure 5(a);

Figure 6 is a vertical section of a tank suitable for containing an aquatic organism of the Biomphalaria type; and

Figure 7 is a vertical section of a tank suitable for containing an aquatic organism of the Brachydanio type.

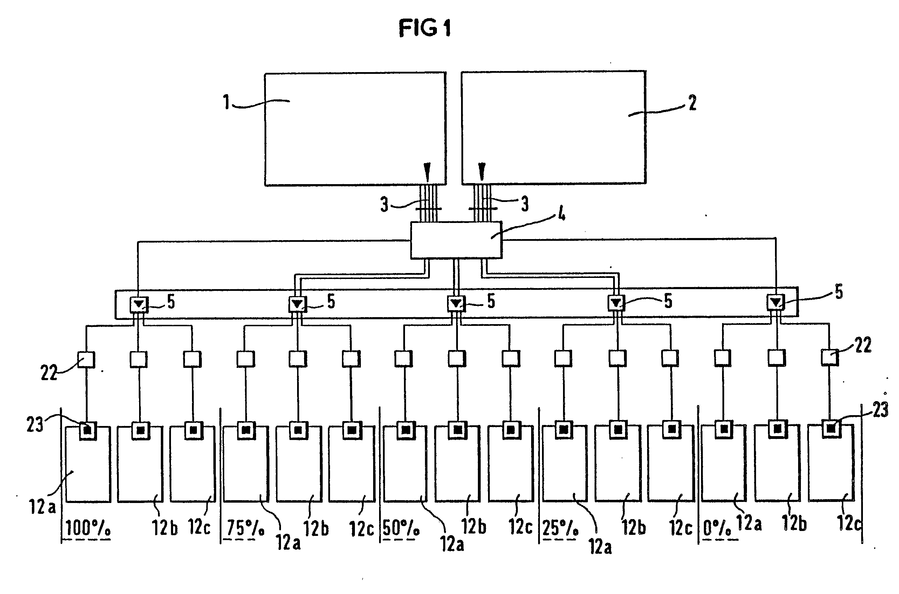

Referring firstly to Figure 1, the diagram is divided into three sections: an upper section in which the desired dilution of toxic liquid takes place; a middle section, in which the solutions of various concentrations of toxic substance are separated and distributed to the several tanks containing the different species of organism; which are in the bottom section of the diagram.

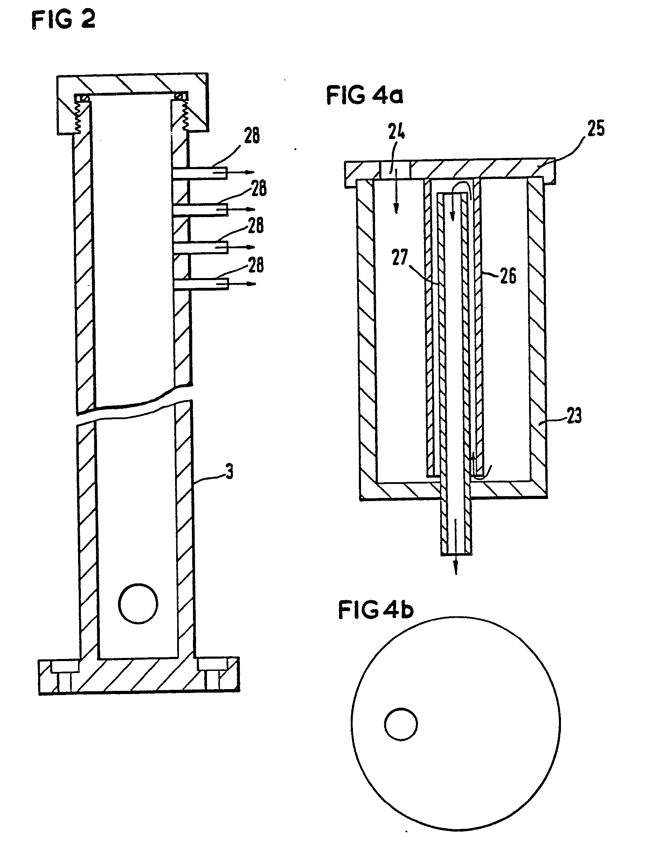

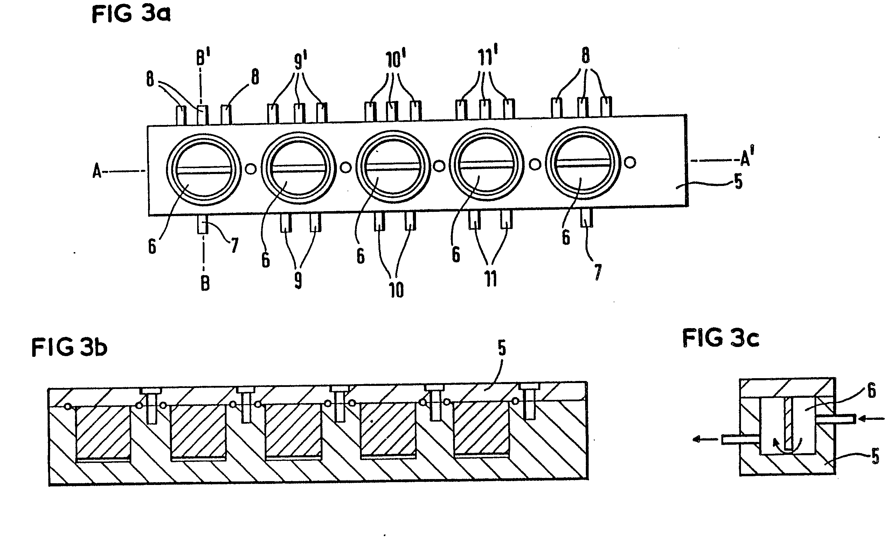

In the upper section of the diagram there is the tank 1 containing the original solution of the toxic substance and tank 2 for the unpolluted dilution water. Each of these two tanks are connected to its own header 3 (Figure 2). Each header is in turn connected to a peristaltic pump 4 where the dilution of the original solution of substance to the desired concentration of solution is obtained by means of a set of tubes with different diameters.

Referring to Figure 2, each header, which has a cylindrical form, is fed at the bottom respectively with water or toxic solution. Four exit tubes 28, in the upper part of the header permit the flow of the liquid to the peristaltic pump.

The solution to be tested and the dilution water flow into a mixing unit or diffuser 5, shown in Figures 3(a), 3(b) and 3(c), and shown schematically in Figure 1 by reference numeral 5, for the five different concentrations. In Figure 3 are shown the diffuser 5 composed of five mixing chambers 6 in which water and toxic solution are mixed according to the desired selected concentration. The mixing chambers placed at the extremities of the diffuser 5 have only one entry tube 7, because they are intended respectively for the full concentration of 100% of original toxic solution or only water (0% concentration of original toxic solution). The three exit tubes 8 are for the three species of living organism to be studied in 100% concentration and water respectively. The pairs of entry tubes 9, 10 and 11 of the other mixing chambers 6, coming from the peristaltic pump, serve to mix the tonic solution and dilution water at 75%, 50% and 25% respectively of concentration of toxic substance in said chamber 6 and, through the three exit tubes, respectively 9', 10' and 11' solutions of said concentrations of original toxic solution, regulated by electrovalves 22, feed the tanks 12(a), 12(b) and 12(c) containing respectively the three species of organism. As shown in the bottom section of Figure 1, there are five series of tanks corresponding to the five different concentrations of toxic solution, each series comprising three types of container for the three respective types of organisms. Before entering the tanks 12(a), 12(b) and 12(c) and for a better and more homogeneous distribution in the tanks, the liquid passes through a siphon 23.

The flow of the liquid from the exit tubes 8, 9', 10' and 11' and directed to the tanks 12(a), 12(b) and 12(c) is controlled by electrovalves 22 which normally are in the off position. The operating time of each electrovalve is adjusted in relation to the quantity of liquid that must reach the tanks 12(a), 12(b) and 12(c) in relation to the volume needed for a complete turnover of the solution. In fact, the replacement volume varies with the volume and the form of each type of tank. The valves are actuated by three sequentially operating timers, for simplicity not shown.

Referring to Figures 4(a) and 4(b), the siphons, one for each tank, are fed at the top through an eccentrically disposed hole 24 in the cover 25. Each siphon contains two concentric vertical cylinders 26 and 27 through which the liquid flows and enters the tanks 12 (a), 12(b) and 12(c) only when the siphon has been completely filled. The direction of liquid flow through the siphon is shown by arrows.

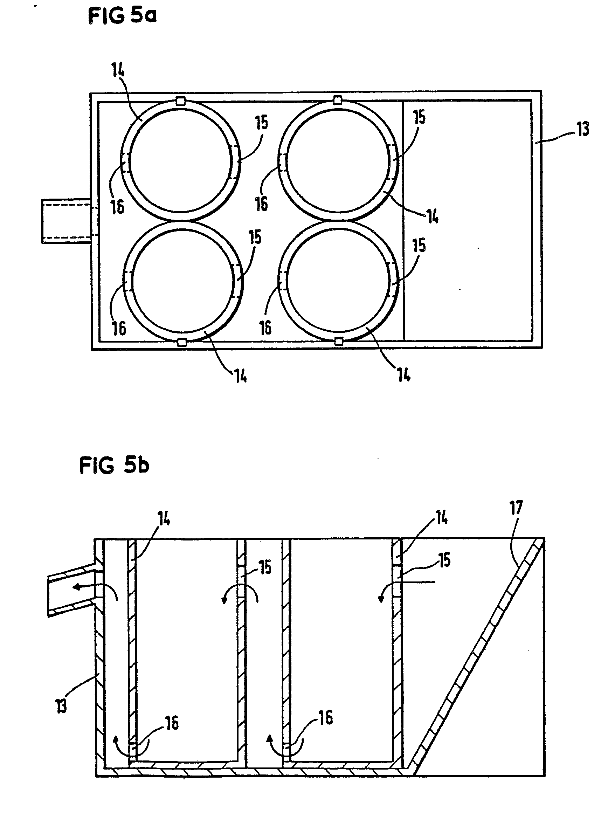

A tank 13 suitable for Daphnia is shown in Figures 5(a) and 5(b) in horizontal and vertical planes respectively. Each tank 13 contains a double pair of cylinders 14, into which the organisms to be tested are introduced. Two holes, diametrically opposite, one, the larger 15, in the upper and the other 16 in the lower part of each cylinder, channel the flow of the toxic solution into each cylinder and prevent the organisms from reaching the surface. For better regulation of the flow, the solution enters the tank by lapping an inclined wall 17. Furthermore, the four cylinders used in the case of Daphnia, permit four replicated samples to be obtained for each test, and this overcomes one of the main difficulties in interpreting toxicity tests on this species.

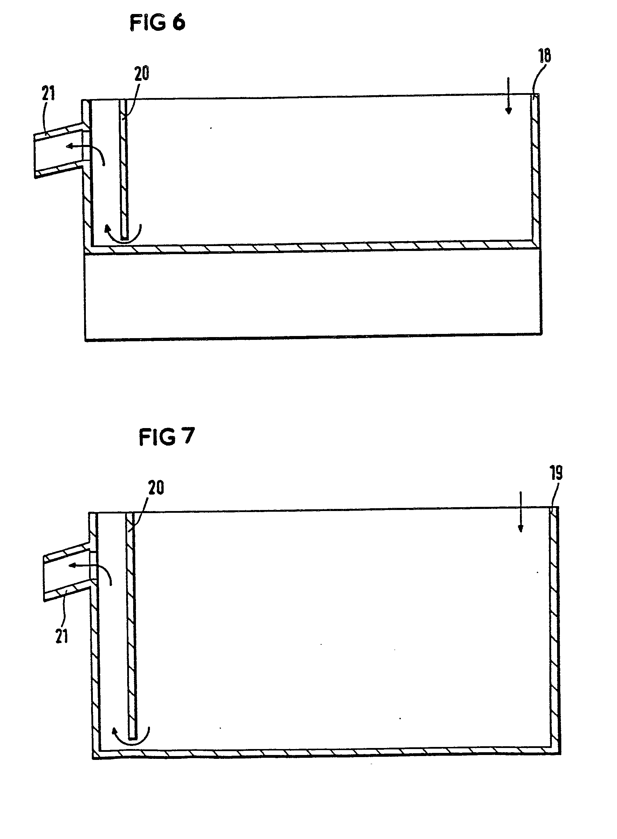

The tanks 18 for Biomphalaris and 19 for Brachydanio, shown in vertical section in Figures 6 and 7, respectively, are similar in form but different in volume, owing to the different requirements of the two species. Both types of tanks are fitted internally with a vertical wall 20, parallel to one side and near the exit 21 of the tanks, which leaves a passage only a few millimeters high above the bottom of the tank. This wall facilitates the mixing of the solution into the tank, inasmuch as it compels the liquid to flow first from the top down to the bottom, then, during the outflow, from the bottom up to the top. Obviously, the form of the three types of tanks can be modified, in relation to the requirements of other types of organisms which may be tested in the apparatus of the invention.

All of the tanks are placed in a water bath (not shown) at constant temperature to provide uniform conditions during the tests.

The apparatus as hereinbefore described is not limited to the examples given, but may be employed for example for analysing toxic soluble substances, single and mixed, polluted water or rivers and lakes and industrial waste water.

In the embodiment described there may be studied in continuous flow and in the same operative conditions, for example three different types of organisms in four different concentrations of toxic substance, or two different types of organisms in six different concentrations of toxic concentrations of toxic substance.

The flexibility of the apparatus permits also the variation of the choice of the concentration of toxic substance by varying the speed of the peristaltic pump and of the diameter of the tubes of the peristaltic pump.

As will be readily appreciated by those skilled in the art other concentrations and different types and numbers of organism may be used in tests with the apparatus of the invention.

Furthermore the characteristics of the apparatus are such that it may be easily used both in the laboratory and in the field.

## Claims

1. An apparatus for measuring the toxicity of pollutants to aquatic living organisms in which the effect of a presumed toxic substance on aquatic living organisms is measured at different concentrations of the toxic substance characterised in that the apparatus is adapted to measure the effects on one or more organisms simultaneously in a continuous liquid flow, the apparatus comprising a tank (1) for storing the toxic substance in liquid form and a tank (2) for storing unpolluted dilution water, two headers (3) connecting the respective tanks to a peristaltic pump (4), the pump being adapted by having flexible tubes (9, 10, 11, 9', 10', 11') of different diameters simultaneously to supply preselected, differently-proportioned amounts of toxic liquid and dilution water, and means (5) for mixing and distributing said differently proportioned amounts of toxic liquid and dilution water through siphons to tanks (12a, 12b, 12c) containing the organisms to be tested.

2. An apparatus as claimed in claim 1 wherein the flow of the mixture of toxic liquid and dilution water for each species of organism to be tested is regulated by electrovalves (22) controlled by timers.

3. An apparatus as claimed in claim 1 or claim 2 which is adapted to measure the effect of a toxic liquid at a plurality of concentrations on a plurality of different organisms.

4. An apparatus as claimed in claim 1 or claim 2 adapted to test one species of organism simultaneously at seven different concentrations of toxic liquid and a control reference of dilution water.

5. An apparatus as claimed in any one of claims 1 to 3 which is adapted to test two different species of organism simultaneously at six different concentrations of toxic liquid and a control reference of dilution water.

6. An apparatus as claimed in any one of claims 1 to 3 which is adapted to test three different species simultaneously at four different concentrations of toxic liquid and a control reference of dilution water.

7. A method of measuring the toxicity of pollutants to aquatic living organisms which method comprises operating an apparatus as claimed in any one of the preceding claims and observing the effects on the organisms in the tanks containing them.

## Revendications

1. Appareil de mesure de la toxicité de polluants vis-à-vis d'organismes aquatiques vivants, dans lequel l'effet d'un substance toxique présumée sur des organismes aquatiques vivants est mesuré à différentes concentrations de la substance toxique, caractérisé en ce que l'appareil est destiné à mesurer simultanément les effets, sur un ou plusieurs organismes, dans un courant continu de liquide, l'appareil comprenant un réservoir (1) destiné à conserver la substance toxique sous forme liquide et un réservoir (2) destiné à conserver de l'eau non polluée de dilution, deux collecteurs (3) reliant les réservoirs respectifs à une pompe péristaltique (4), la pompe étant adaptée, par des tubes souples (9, 10, 11, 9', 10', 11') de diamètres différents, à la transmission simultanée de quantités prédéterminées et en proportion différente de liquide toxique et d'eau de dilution, et un dispositif (5) de mélange et de distribution des quantités de liquide toxique et d'eau de dilution en proportion différente par l'intermédiaire de siphons à des réservoirs (12a, 12b, 12c) contenant les organismes à tester.

2. Appareil selon la revendication 1, dans lequel le courant du mélange de liquide toxique et d'eau de dilution, pour chaque espèce d'organisme à tester, est régulé par des électrovannes (22) commandées par des minuteries.

3. Appareil selon l'une des revendications 1 et 2, adapté à la mesure de l'effet d'un liquide toxique à plusieurs concentrations sur plusieurs organismes différents.

4. Appareil selon l'une des revendications 1 et 2, adapté à tester une espèce d'organisme simultanément à sept concentrations différentes de liquide toxique avec un témoin d'eau de dilution.

5. Appareil selon l'une quelconque des revendications 1 à 3, adapté à l'essai de deux espèces différentes d'organisme simultanément à six concentrations différentes de liquide toxique, avec un témoin d'eau de dilution.

6. Appareil selon l'une quelconque des revendications 1 à 3, adapté à l'essai de trois espèces différentes simultanément à quatre concentrations différentes de liquide toxique, avec un témoin d'eau de dilution.

7. Procédé de mesure de la toxicité de polluants vis-à-vis d'organismes aquatiques vivants, le procédé comprenant la mise en oeuvre d'un appareil selon l'une quelconque des revendications précédentes, et l'observation des effets sur les organismes placés dans les réservoirs qui les contiennent.

## Patentansprüche

1. Vorrichtung zum Messen der Giftigkeit von Verunreinigungen für im Wasser lebende Organismen, bei der die Wirkung einer vermuteten toxischen Substanz auf im Wasser lebende Organismen bei unterschiedlichen Konzentrationen der giftigen Substanz gemessen wird, dadurch gekennzeichnet, daß die Vorrichtung zum gleichzeitigen Messen der Wirkungen auf einen oder mehrere Organismen in einem kontinuierlichen Flüssigkeitsstrom ausgelegt ist, wobei die Vorrichtung aus einem Behälter (1) zur Aufnahme der giftigen Substanz in flüssiger Form und einem Behälter (2) zur Aufnahme von nichtverunreinigtem Verdünnungswasser, zwei Sammlern (3), die die jeweiligen Behälter mit einer peristaltischen Pumpe (4) verbinden, wobei die Pumpe mit biegsamen Rohren (9, 10, 11, 9', 10', 11') von unterschiedlichen Durchmessern zu gleichzeitigen Zuführen von vorgewählten, unterschiedlich proportionierten Mengen an giftiger Flüssigkeit und Verdünnungswasser versehend ist, und Mitteln (125) zum Mischen und Verteilen dieser unterschiedlich porportionierten Mengen an giftiger Flüssigkeit und Verdünnungswasser durch Saugheber zu Behältern (12a, 12b, 12c), die die zu prüfenden Organismen enthalten, besteht.

2. Vorrichtung nach Anspruch 1, dadurch gekennzeichnet, daß die Gemischströme von giftiger Flüssigkeit und Verdünnungswasser für jede Art zu prüfendem Organismus durch durch Zeitgeber gesteuerte Elektroventile (22) reguliert werden.

3. Vorrichtung nach Anspruch 1 oder 2, dadurch gekennzeichnet, daß sie zum Messen der Wirkung einer giftigen Flüssigkeit bei einer Vielzahl von Konzentrationen auf eine Vielzahl von unterschiedlichen Organismen ausgelegt ist.

4. Vorrichtung nach Anspruch 1 oder 2, dadurch gekennzeichnet, daß sie zum Prüfen von einer Organismusart gleichzeitig bei sieben unterschiedlichen Konzentrationen von giftiger Flüssigkeit und einer Vergleichsprobe von Verdünnungswasser ausgelegt ist.

5. Vorrichtung nach einem der Ansprüche 1 bis 5, dadurch gekennzeichnet, daß sie zur Prüfung von zwei unterschiedlichen Arten von Organismen gleichzeitig bei sechs unterschiedlichen Konzentrationen von giftiger Flüssigkeit und einer Vergleichsprobe von Verdünnungswasser ausgelegt ist.

6. Vorrichtung nach einem der Ansprüche 1 bis 3, dadurch gekennzeichnet, daß sie zum Prüfen von drei unterschiedlichen Arten gleichzeitig bei vier unterschiedlichen Konzentrationen von giftiger Flüssigkeit und einer Vergleichsprobe von Verdünnungswasser ausgelegt ist.

7. Verfahren zum Messen der Giftigkeit von Verunreinigungen für im Wasser lebende Organismen, dadurch gekennzeichnet, daß eine Vorrichtung nach einem der vorhergehenden Ansprüche betrieben und die Wirkungen auf die Organismen in den sie enthaltenden Behältern beobachtet werden.

FIG 1

**FIG 2**

**FIG 4a**

**FIG 4b**

FIG 3a

FIG 3b

FIG 3c

## FIG 5a

## FIG 5b

0 045 623

FIG 6

FIG 7